# EUROPEAN PATENT APPLICATION

(11) **EP 1 911 492 A1**
(43) Date of publication of application: **16.04.2008**
(21) Application number: 07253956.2
(22) Date of filing: 05.10.2007
(51) Int. Cl.: A61N 1/37, A61B 5/046, A61N 1/362

(54) **System and related methods for monitoring cardiac disease using pacing latency measurements**

(30) Priority: 09.10.2006 US 539824
(71) Applicant: PACESETTER, INC., Sylmar, CA 91342-9221 (US)
(72) Inventor: Min, Xiaoyi, Thousand Oaks, CA 91362 (US); Gill, Jong, Valencia, CA 91355 (US)
(74) Representative: Rees, David Christopher

(57) **Abstract**

A system and related methods for monitoring cardiac disease. The system includes an implantable medical device having a microcontroller that is configured to measure a pacing latency value for a region of a heart, compare the measured pacing latency value to a previously measured pacing latency value for the region of the heart, and determine a change in an amount of cardiac disease in the region of the heart based on the comparison of the measured pacing latency values.

## Description

The invention relates to the field of implantable medical devices ("IMDs"). More specifically, the invention relates to a system and related methods for monitoring cardiac disease.

IMDs, for example, bradycardia and antitachycardia pacemakers, defibrillators, and cardioverters, are surgically implanted into a patient and configured to stimulate a patient's heart muscles by delivering electrical pulses, via electrodes, to the heart in response to measured cardiac events. IMDs also are configured to monitor the patient's heart.

As part of an IMD's efforts to monitor the patient's heart, the IMD can monitor heart failure surrogates, *i*.*e*., indicators of the heart's physical condition. Examples of heart failure surrogates include the following: exercise compliance, *i.e.*, the patient's conformity in fulfilling their exercise therapy obligations; heart rate variability, *i*.*e*., the beat-to-beat alteration in heart rate; heart rate trend, *i*.*e*., a prevailing tendency of the heart's rate; heart rate recovery, *i.e.*, a measurement of how quickly the heart rate drops after a peak in exercise; DC impedance, *i*.*e*., the DC impedance measurement of the heart using a combination of two electrodes; left atrial pressure, *i.e.*, the blood pressure within the left atrium; and evoked response, *i*.*e*., the hearts response to an electrical stimulus applied by an IMD electrode to the myocardium, typically a peak-to-peak amplitude value or a slope value.

However, monitoring heart failure surrogates can be costly in terms of financial costs, the time it takes to do the various studies, and a multiplicity of external complex technologies such as Doppler-echocardiography, magnetic resonance imaging, radiography, and a variety of chemical tests that are used to evaluate kidney and hepatic function reflecting cardiac perfusion, all of which need to be scheduled and the results of which are not usually immediately available. Additional sensors also are being incorporated into implanted pulse generators, for example, sensors that are used to monitor heart rate variability, which requires increased computing power; and sensors that are used to monitor lung water, which also requires computing power combined with the injection of low amplitude pulses on a frequent basis to measure impedance. These requirements increase the complexity of these devices as well as their costs and the time that is required by the medical practitioner to retrieve the data. Also, various heart failure surrogates, for example, the evoked response and DC impedance measurements values can be influenced by the patient's posture during the measurement. While monitoring heart failure, be it by surrogates or directly, can be costly and difficult to measure, monitoring heart failure is essential in order to allow clinical intervention in a timely manner, to minimize hospitalization of the patient, and to improve the patient's overall quality of life.

It should, therefore, be appreciated that there is a need for a cost-efficient system and methods for monitoring cardiac disease using an IMD, where the measurements that are performed by the IMD are not influenced by the patient's posture. The present invention satisfies these needs.
According to the invention, there is provided an implantable medical device comprising: a microcontroller that is adapted to measure a pacing latency value in a region of a heart, and to determine a cardiac disease condition in the region of the heart based on a comparison of the measured pacing latency value to a previously measured pacing latency value.
Preferably, the microcontroller is configured to measure a heart failure surrogate value, and to characterise an amount of cardiac disease for the heart based on a combination of the measured heart failure surrogate value and the comparison of the measured pacing latency values. Preferably, the implantable medical device is configured to notify a medical practitioner of the change in the amount of cardiac disease in the region of the heart. Preferably, the medical practitioner is notified of the change in the amount of cardiac disease in the region of the heart when an absolute change in the measured pacing latency value or a relative change in the measured pacing latency value is greater than or equal to a predetermined threshold value.
The invention also extends to a system comprising means for measuring a pacing latency value for a region of a heart; means for comparing the measured pacing latency value to a previously measured pacing latency value for the region of the heart; and means for determining a cardiac disease condition based on the comparison of the measured pacing latency values. The system may include means for generating a notification signal of the cardiac disease condition.
The invention also extends to a method for monitoring cardiac disease, the method comprising:
a) measuring a pacing latency value for a region of a heart;
b) comparing the measured pacing latency value to a previously measured pacing latency value for the region of the heart; and
c) determining a cardiac disease condition based on the comparison of the measured pacing latency values.
Preferably, the previously measured pacing latency value is calculated from an average of a plurality of previously measured latency values. The method may further comprise reconfirming the measured pacing latency value if the measured pacing latency value is different from the previously measured pacing latency value. The method may, further comprise notifying a medical practitioner of the change of cardiac disease.
Preferably, the step a) of measuring the pacing latency value for the region of the heart includes:
i) measuring a first pacing latency value for an atrium, and
ii) measuring a second pacing latency value for a ventricle;
the step b) of comparing the measured pacing latency value to the previously measured pacing latency value for the region of the heart includes:
i) comparing the measured first pacing latency value to a previously measured first pacing latency value for the atrium, and
ii) comparing the measured second pacing latency value to a previously measured second pacing latency value for the ventricle; and the step c) of determining the change in the amount of cardiac disease in the region of the heart based on the comparison of the pacing latency values includes determining a change in the amount of cardiac disease in the region of the heart based on both the comparison of the first pacing latency values for the atrium and the comparison of the second pacing latency values for the ventricle.
The method may further comprise:
measuring a heart failure surrogate value; and
characterising an amount of cardiac disease for the heart based on a combination of the measured heart failure surrogate value and the comparison of the pacing latency values. Preferably, the step a) of measuring the pacing latency value for the region of the heart includes: i) measuring a first pacing latency value for a left ventricle, and ii) measuring a second pacing latency value for a right ventricle; the step b) of comparing the measured pacing latency value to the previously measured pacing latency value for the region of the heart includes: i) comparing the measured first pacing latency value to a previously measured first pacing latency value for the left ventricle, and ii) comparing the measured second pacing latency value to a previously measured second pacing latency value for the right ventricle; and the step c) of determining the change in the amount of cardiac disease in the region of the heart based on the comparison of the pacing latency values includes determining a change in ventricular remodelling based on both the comparison of the first pacing latency values and the comparison of the second pacing latency values.
The region of the heart may be selected from the group consisting of a left atrium, a left ventricle, a right atrium, and a right ventricle.

Certain embodiments described herein relate to a system for monitoring cardiac disease in a cost-efficient manner using an IMD. The measurements that are performed by the IMD are not influenced by the patient's posture during the measurement. An exemplary method for monitoring cardiac disease includes measuring a pacing latency value for a region of a heart, comparing the measured pacing latency value to a previously measured pacing latency value for the region of the heart, and determining a change in an amount of cardiac disease in the region of the heart based on the comparison of the measured pacing latency values.

Other features of the invention should become apparent from the following description of the preferred embodiments taken in conjunction with the accompanying drawings, which illustrate, by way of example, the principles of the invention.

Fig. 1 is a simplified diagram illustrating an IMD embodying the present invention, which is electrically coupled to three leads that are positioned within a patient's heart;

Fig. 2 is a functional block diagram of the IMD of Fig. 1;

Fig. 3 is a simplified diagram illustrating a programmer control system, which is configured to communicate with the IMD of Fig. 1;

Fig. 4 is a simplified diagrammatic illustration of an example of a P-QRS-T complex as recorded with a surface electrocardiogram ("ECG");

Fig. 5 is a printout of a surface ECG that demonstrates latency from an atrial stimulus to an onset of visible atrial depolarization;

Fig. 6 is a simplified diagram that demonstrates the timing of an evoked response signal where a standard sensing circuit is absolutely refractory and a separate dedicated detection circuit for the sensing evoked response is operating;

Fig. 7 is a printout of an intracardiac ECG that demonstrates latency from an atrial stimulus to an intracardiac electrical depolarization in the atrium; and

Fig. 8 is a flow diagram of an algorithm according to the present invention, which can be implemented by the IMD of Fig. 1.

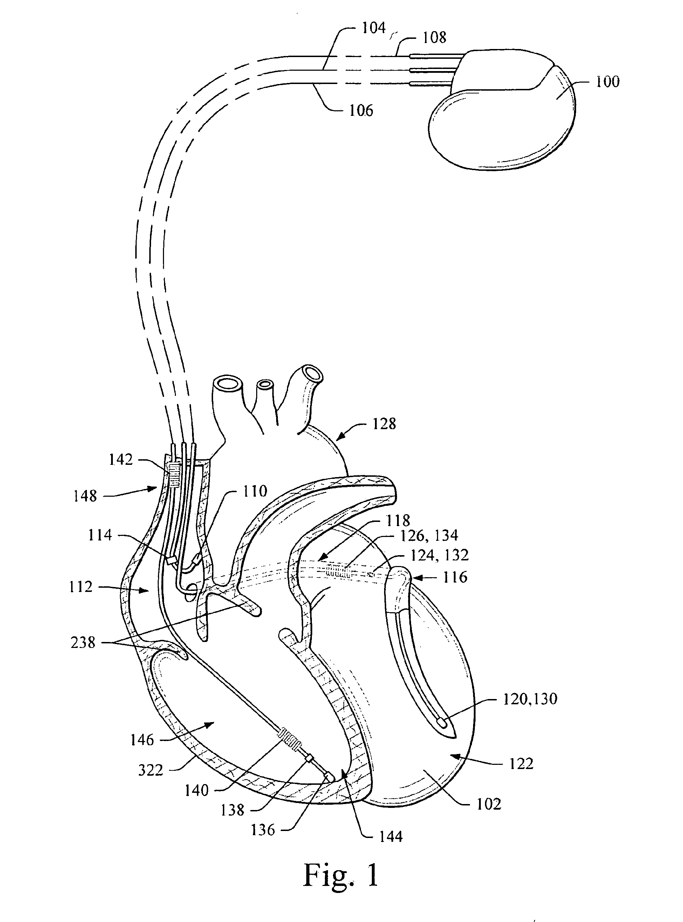

Although the invention can be used in conjunction with a wide variety of IMDs, with reference now to the illustrative drawings, and particularly to Fig. 1, there is shown an exemplary IMD 100, a heart stimulation device, in electrical communication with a patient's heart 102 by way of three leads 104, 106, and 108, suitable for delivering multi-chamber stimulation and shock therapy. To sense atrial cardiac signals and to provide right atrial chamber stimulation therapy, the IMD is coupled to an implantable right atrial lead 104 having at least an atrial tip electrode 110, which typically is implanted in contact with the patient's right atrium 112, *e.g.*, the right atrial appendage. As shown in Fig. 1, the right atrial lead 104 also includes a right atrial ring electrode 114.

To sense left atrial and ventricular cardiac signals and to provide left chamber pacing therapy, the IMD 100 is coupled to a coronary sinus lead 106, which is designed for placement in the coronary sinus region 116 via the coronary sinus 118, and for positioning a distal electrode 120 adjacent to the left ventricle 122 and/or additional electrode(s) 124 and 126 adjacent to the left atrium 128. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein, or any other cardiac vein accessible by the coronary sinus.

Accordingly, an exemplary coronary sinus lead 106 is designed to receive atrial and ventricular cardiac signals and to deliver left ventricular pacing therapy using at least a left ventricular tip electrode 130, left atrial pacing therapy using at least a left atrial ring electrode 132, and shocking therapy using at least a left atrial coil electrode 134. For a complete description of a coronary sinus lead, the reader is directed to U.S. Patent No. 5,466,254, entitled "Coronary Sinus Lead with Atrial Sensing Capability" to Helland; which is incorporated by reference herein.

In Fig. 1, the IMD 100 also is shown in electrical communication with the patient's heart 102 by way of an implantable right ventricular lead 108 having, in this implementation, a right ventricular tip electrode 136, a right ventricular ring electrode 138, a right ventricular coil electrode 140, and a superior vena cava ("SVC") coil electrode 142. Typically, the right ventricular lead is transvenously inserted into the heart to place the right ventricular tip electrode in the right ventricular apex 144 so that the right ventricle coil electrode will be positioned in the right ventricle 146 and the SVC coil electrode will be positioned in the superior vena cava 148. Accordingly, the right ventricular lead is capable of sensing or receiving cardiac signals, and delivering stimulation in the form of pacing and shock therapy to the right ventricle.

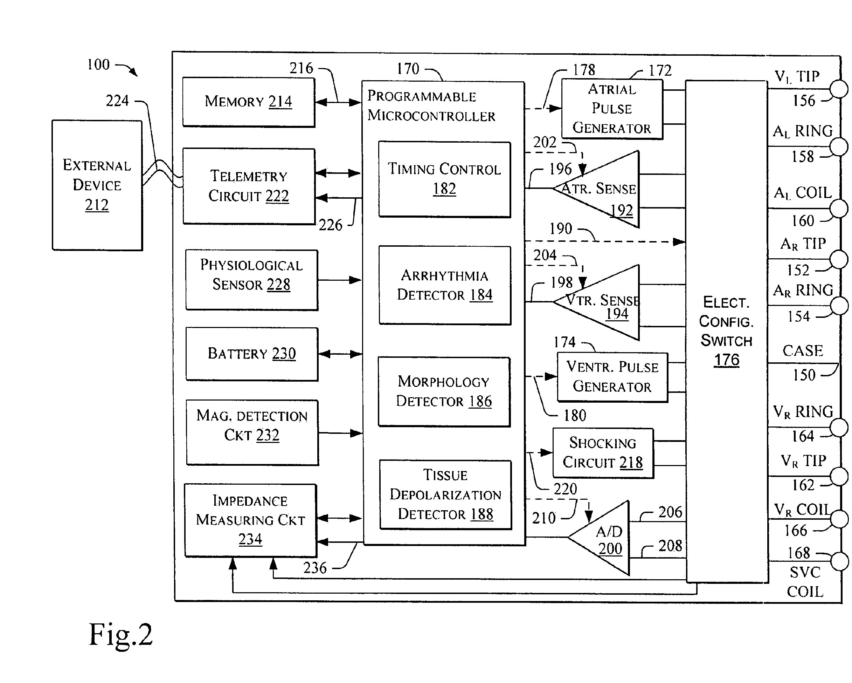

Fig. 2 is an exemplary block diagram that depicts various components of the IMD 100 shown in Fig. 1. The IMD can be configured to treat both fast and slow arrhythmias with stimulation therapy, including cardioversion, defibrillation, and pacing stimulation. While a particular multi-chamber device is shown in Fig. 1, it is to be appreciated and understood that this is done for illustration purposes only. Thus, the techniques and methods described below can be implemented in connection with any suitably configured or configurable heart stimulation device. Accordingly, one of skill in the art could readily duplicate, eliminate, or disable the appropriate circuitry in any desired combination to provide a heart stimulation device that is capable of treating the appropriate chamber(s) 112, 122, 128, and 146 with cardioversion, defibrillation, and pacing stimulation.

The IMD 100 includes a housing 150, which often is referred to as the "can", "case", or "case electrode", and can be selected programmably to act as the return electrode for all "unipolar" modes of operation for the IMD. The housing can further be used as a return electrode alone, or in combination with one or more of the coil electrodes 134, 140 and 142 for shocking purposes. The housing further includes a connector (not shown) having a plurality of terminals 152-168 (shown schematically and, for convenience, the names of the electrodes to which they are connected are shown next to the terminals).

To achieve right atrial sensing and pacing, the connector (not shown) includes at least a right atrial tip terminal ("A_{R} TIP") 152 adapted for coupling to the atrial tip electrode 110. As shown in Fig. 2, the block diagram also includes a right atrial ring terminal ("A_{R} RING") 154 adapted for coupling to the atrial ring electrode 114. To achieve left chamber sensing, pacing, and shocking, the connector includes at least a left ventricular tip terminal ("V_{L} TIP") 156, a left atrial ring terminal ("A_{L} RING") 158, and a left atrial shocking terminal ("A_{L} COIL") 160, which are adapted for coupling to the left ventricular tip electrode 130, the left atrial ring electrode 132, and the left atrial coil electrode 134, respectively.

To support right chamber sensing, pacing, and shocking, the connector (not shown) further includes a right ventricular tin terminal ("V_{R} TIP") 162, a right ventricular ring terminal ("V_{R} RING") 164, a right ventricular shocking terminal ("V_{R} COIL") 166, and a superior vena cava shocking terminal ("SVC COIL") 168, which are adapted for coupling to the right ventricular tip electrode 136, right ventricular ring electrode 138, the right ventricle coil electrode 140, and the SVC coil electrode 142, respectively.

The IMD 100 further includes a programmable microcontroller 170, a microprocessor-based control circuit, which controls the various modes of stimulation therapy. As is well known in the art, a microcontroller typically includes a microprocessor, or equivalent control circuitry, designed specifically for controlling the delivery of stimulation therapy, and can further include random access memory ("RAM") or read-only memory ("ROM"), logic and timing circuitry, state machine circuitry, and input/output ("I/O") circuitry. The microcontroller generally includes the ability to process or monitor input signals (data or information) as controlled by a program code stored in a designated block of memory. The type of microcontroller included in the IMD is not critical to the described implementations; hence, any suitable microcontroller can be used that carries out various functions such as those described herein. The use of microcontrollers for performing timing and data analysis functions are well known in the art.

Representative types of control circuitry that may be used in connection with the described embodiments can include the microprocessor-based control system of U.S. Patent No. 4,940,052 to Mann et al., the state machine of U.S. Patent No. 4,712,555 to Thornander et al., and U.S. Patent No. 4,944,298 to Sholder, all of which are incorporated by reference herein. For a more detailed description of the various timing intervals used within the IMD 100 and their interrelationship, see U.S. Patent 4,788,980 to Mann et al., also incorporated by reference herein.

Fig. 2 also shows an atrial pulse generator 172 and a ventricular pulse generator 174, which are coupled to the microcontroller 170, and which generate pacing stimulation pulses for delivery by the right atrial lead 104, the coronary sinus lead 106, and/or the right ventricular lead 108 via an electrode configuration switch 176. It is understood that in order to provide stimulation therapy in each of the four chambers 112, 122, 128, and 146 of the heart 102, the atrial and the ventricular pulse generators can include dedicated, independent pulse generators, multiplexed pulse generators, or shared pulse generators. The atrial and ventricular pulse generators are controlled by the microcontroller via appropriate control signals 178 and 180, respectively, which trigger or inhibit the stimulation of pulses by the pulse generators.

The microcontroller 170 further includes timing control circuitry 182 that is configured to control the timing of the stimulation pulses, *e.g.*, pacing rate, atrio-ventricular ("A-V") delay, atrial interconduction ("A-A") delay, or ventricular interconduction ("V-V") delay, etc., as well as to keep track of the timing of refractory periods, blanking intervals, noise detection windows, evoked response windows, alert intervals, marker channel timing, etc., which are well known in the art.

The microcontroller 170 can further include arrhythmia detector circuitry 184, morphology detector circuitry 186, and tissue depolarization detector circuitry 188. These components can be utilized by the IMD 100 when determining desirable times to administer various therapies. The components 184-188 can be implemented in hardware as part of the microcontroller, or as software/firmware instructions programmed into the IMD and executed by the microcontroller during certain modes of operation.

The electrode configuration switch 176 includes a plurality of switches for coupling the desired electrodes 110, 114, and 130-142 to the appropriate I/O circuits, *e.g.*, the atrial and ventricular pulse generators 172 and 174, respectively, thereby providing complete electrode programmability. Accordingly, the electrode configuration switch, in response to a control signal 190 from the microcontroller 170, determines the polarity of the stimulation pulses, *e.g.*, unipolar, bipolar, combipolar, etc., by selectively closing the appropriate combination of switches, which are included in the electrode configuration switch, as is known in the art.

Atrial sensing circuits 192 and ventricular sensing circuits 194 also can be selectively coupled to the right atrial lead 104, the coronary sinus lead 106, and/or the right ventricular lead 108, through the electrode configuration switch 176 for detecting the presence of cardiac activity in each of the four chambers 112, 122, 128, and 146 of the heart 102. Accordingly, the atrial sensing circuit and the ventricular sensing circuit can include dedicated sense amplifiers, multiplexed amplifiers, or shared amplifiers. The electrode configuration switch determines the "sensing polarity" of the cardiac signal by selectively closing the appropriate switches, which are included in the electrode configuration switch, as is also known in the art. In this way, the medical practitioner can program the sensing polarity independent of the stimulation polarity. The sensing circuits, *e.g.*, the atrial and ventricular sensing circuits, are optionally capable of obtaining information indicative of tissue depolarization.

Each atrial and ventricular sensing circuit 192 and 194, respectively, preferably employs one or more low-power, precision amplifiers with programmable gain and/or automatic gain control, bandpass filtering, and a threshold detection circuit, as is known in the art, to selectively sense the cardiac signal of interest. The automatic gain control enables the IMD 100 to deal effectively with the difficult problem of sensing the low-amplitude signal characteristics of atrial or ventricular fibrillation. For a complete description of a typical sensing circuit, the reader is directed to U.S. Patent No. 5,573,550 ("the '550 patent"), entitled "Implantable Stimulation Device having a Low-Noise, Low-Power, Precision Amplifier for Amplifying Cardiac Signals" to Zadeh et al. For a complete description of an automatic gain control system, the reader is directed to U.S. Patent No. 5,685,315 ("the '315 patent"), entitled "Cardiac Arrhythmia Detection System for an Implantable Stimulation Device" to McClure et al.

The outputs 196 and 198 of the atrial and ventricular sensing circuits 192 and 194, respectively, are coupled to the microcontroller 170, which, in turn, is able to trigger or inhibit the atrial and ventricular pulse generators 172 and 174, respectively, in a demand fashion in response to the absence or presence of cardiac activity in the appropriate chambers 112, 122, 128, and 146 of the heart 102. Furthermore, the microcontroller is also capable of analyzing information output from the atrial and ventricular sensing circuits, and/or an analog-to-digital ("A/D") data acquisition system 200 (the A/D data acquisition system is discussed below) to determine, or detect, whether, and to what degree, tissue depolarization has occurred in the heart and to program a pulse, or pulses, in response to such determinations. The atrial and ventricular sensing circuits, in turn, receive control signals over signal lines 202 and 204, respectively, from the microcontroller for purposes of controlling the gain, threshold, polarization charge removal circuitry (not shown), and the timing of any blocking circuitry (not shown) coupled to the inputs of the atrial and ventricular sensing circuits, as is known in the art.

For arrhythmia detection, the IMD 100 utilizes the atrial and ventricular sensing circuits 192 and 194, respectively, which are coupled between the microcontroller 170 and the electrode configuration switch 176, and configured to sense cardiac signals to determine whether a rhythm is physiologic or pathologic. In reference to arrhythmias, as used herein, "sensing" is reserved for the noting of an electrical signal or obtaining data (information), and "detection" is the processing (analysis) of these sensed signals and noting the presence of an arrhythmia. Of course, a circuit can accomplish both sensing and detection simultaneously. In addition, such a circuit also can ascertain an event cycle length as well. The timing intervals between sensed events, *e.g.*, P-waves, R-waves, and depolarization signals associated with fibrillation which are sometimes referred to as "F-waves" or "Fib-waves", are then classified by the arrhythmia detector 184 included in the microcontroller by, for example, comparing them to a predefined rate zone limit, *i.e.,* bradycardia, normal, low-rate ventricular tachycardia ("VT"), high-rate VT, and fibrillation rate zones, and/or various other characteristics, *e.g.*, sudden onset, stability, physiologic sensors, and morphology, etc. Such classification can aid in the determination of the type of remedial therapy that is needed, *e.g.*, bradycardia pacing, anti-tachycardia pacing, cardioversion shocks or defibrillation shocks, collectively referred to as "tiered therapy". An arrhythmia cycle length optionally is ascertained during and/or after arrhythmia sensing and/or detection using the same and/or other components.

Cardiac signals are also applied to inputs 206 and 208 of the A/D data acquisition system 200, which is coupled between the microcontroller 170 and the electrode configuration switch 176. The A/D data acquisition system receives control signals over signal line 210 from the microcontroller. The A/D data acquisition system is configured to acquire intracardiac electrogram signals, convert the raw analog data into a digital signal, and/or store the digital signals for later processing and/or telemetric transmission to an external device 212, *e.g.*, a programmer, transtelephonic transceiver, or a diagnostic system analyzer, (hereinafter referred to as a "programmer") which is configured to be operated by the medical practitioner, and to communicate with the IMD 100. The A/D data acquisition system is coupled to the right atrial lead 104, the coronary sinus lead 106, and the right ventricular lead 108 through the electrode configuration switch to sample cardiac signals across any pair of desired electrodes 110, 114, and 130-142.

Advantageously, the A/D data acquisition system 200, or other system or circuitry, *e.g.*, the atrial sensing circuitry 192 and the ventricular sensing circuitry 194, can be coupled to the microcontroller 170, or other detection circuitry, for analyzing the obtained information to detect an evoked response from the heart 102 in response to an applied stimulus, thereby aiding in the detection of local tissue depolarization and/or global tissue depolarization, *i*.*e*., "capture." Global tissue depolarization or capture generally corresponds with contraction of cardiac tissue. For example, the microcontroller is capable of analyzing obtained information to detect a depolarization signal during a window following a stimulation pulse, the presence of which typically indicates that some degree of tissue depolarization has occurred.

To facilitate detection of tissue depolarization, the microcontroller 170 includes a dedicated tissue depolarization detector 188, implemented in hardware and/or software. The tissue depolarization detector is capable of analyzing information obtained through the atrial and ventricular sensing circuits 192 and 194, respectively, and/or the A/D data acquisition system 200. The tissue depolarization detector analyzes the sensed information to produce a result, such as, activation time. Of course, the tissue depolarization detector is also capable of noting whether activation has occurred during any given time period. The tissue depolarization detector or other microprocessor features can use these results to determine pacing pulse regimens and/or other actions. As described herein, the tissue depolarization detector optionally detects local and/or global depolarization.

The implementation of depolarization detection circuitry 188 and algorithms are well known. See, for example, U.S. Patent Nos. 4,729,376 and 4,708,142 to Decote, Jr.; U.S. Patent No. 4,686,988 to Sholder; U.S. Patent No. 4,969,467 to Callaghan et al.; and U.S. Patent No. 5,350,410 to Kleks et al. The depolarization detection circuitry can include special detection circuits, *e.g.*, the detection circuits that are included in the AUTOCAPTURE Pacing System offered by Pacesetter of Sylmar, California, see, for example, U.S. Patent No. 5,350,410 to Kleks et al.

The microcontroller 170 is further coupled to a memory 214 by a suitable data/address bus 216, wherein the programmable operating parameters used by the microcontroller are stored and modified, as required, in order to customize the operation of the IMD 100 to suit the needs of a particular patient. Such operating parameters define, for example, pacing pulse amplitude, pulse duration, electrode polarity, rate, sensitivity, automatic features, arrhythmia detection criteria, and the amplitude, waveshape, and vector of each shocking pulse to be delivered to the patient's heart 102 within each respective tier of therapy. One feature of the described embodiments is the ability to sense and store a relatively large amount of data, *e.g.*, from the A/D data acquisition system 200, which data can then be used for subsequent analysis to guide the programming of the device.

In the case where the IMD 100 is intended to operate as an implantable cardioverter/defibrillator ("ICD") device, the IMD detects the occurrence of an arrhythmia, and automatically applies an appropriate therapy to the heart 102, or aimed at terminating the detected arrhythmia. Various exemplary methods of ICD operation are described below. According to various methods, the microcontroller 170 controls a shocking circuit 218, which is coupled between the microcontroller and the electrode configuration switch 176, by way of a control signal 220. The shocking circuit generates shocking pulses of low energy (up to approximately 0.5 J), moderate energy (from approximately 0.5 J to approximately 10 J), or high energy (from approximately 11 J to approximately 40 J), as controlled by the microcontroller. Such shocking pulses are typically applied to the patient's heart through at least two shocking electrodes, *e.g.*, the left atrial coil electrode 134, the right ventricular coil electrode 140, and/or the SVC coil electrode 142. As noted above, the housing 150 can act as an active electrode in combination with the right ventricular tip electrode 136, or as part of a split electrical vector using the SVC coil electrode or the left atrial coil electrode, *i*.*e*., using the right ventricular tip electrode as a common electrode.

Cardioversion level shocks are generally considered to be of low to moderate energy level, so as to minimize pain felt by the patient, and/or synchronized with an R-wave and/or pertaining to the treatment of tachycardia. Defibrillation shocks are generally of moderate to high energy level, *i.e.*, corresponding to thresholds in the range from approximately 5 J to approximately 40 J, delivered asynchronously, since R-waves may be too disorganized, and pertaining exclusively to the treatment of fibrillation. Accordingly, the microcontroller 170 is capable of controlling the synchronous or asynchronous delivery of the shocking pulses. The term "cardioversion level" and/or "cardioversion", as used herein, include shocks having low, moderate, and high energy levels, *i*.*e*., cardioversion level shocks and defibrillation shocks.

Advantageously, the operating parameters of the IMD 100 can be non-invasively programmed into the memory 214 through a telemetry circuit 222 in telemetric communication via communication link 224 with the programmer 212. The microcontroller 170 activates the telemetry circuit with a control signal 226. The telemetry circuit advantageously allows intracardiac electrograms ("ECGs") and status information relating to the operation of the IMD, as contained in the microcontroller or memory, to be sent to the programmer through the communication link. For examples of such devices, see U.S. Patent No. 4,809,697, entitled "Interactive Programming and Diagnostic System for use with Implantable Pacemaker" to Causey, III et al.; U.S. Patent No. 4,944,299, entitled "High Speed Digital Telemetry System for Implantable Device" to Silvian; and U.S. Patent No. 6,275,734, entitled "Efficient Generation of Sensing Signals in an Implantable Medical Device such as a Pacemaker or ICD" to McClure et al.

The IMD 100 can further include a physiologic sensor 228, commonly referred to as a "rate-responsive" sensor because it typically is used to adjust pacing stimulation rate output from the IMD according to the exercise state of the patient. The physiological sensor can be used to detect changes in cardiac output, changes in the physiological condition of the heart 102, or diurnal changes in activity, *e.g.*, detecting sleep and wake states of the patient. The microcontroller 170 is coupled to the physiological sensor, receives the output of the physiological sensor, and responds by adjusting the various pacing parameters, *e.g.*, rate, A-V Delay, V-V Delay, etc., at which the atrial and ventricular pulse generators 172 and 174, respectively, generate stimulation pulses.

While shown as being included within the IMD 100, it is to be understood that the physiologic sensor 228 also can be external to the IMD, yet still be implanted within, or carried by, the patient. Examples of physiologic sensors that can be implemented in the IMD include known sensors that, for example, sense respiration rate, pH of blood, ventricular gradient, and so forth. Another sensor that can be used is one that detects activity variance, wherein an activity sensor is monitored diurnally to detect the low variance in the measurement corresponding to the sleep state. For a complete description of the activity variance sensor, the reader is directed to U.S. Patent No. 5,476,483 to Bornzin et al., which patent is hereby incorporated by reference.

More specifically, the physiological sensor 228 optionally includes sensors for detecting movement, position, and/or minute ventilation ("MV") in the patient. Minute ventilation is defined as the total volume of air that moves in and out of a patient's lungs in a minute. During use, signals generated by a position sensor and an MV sensor are sent to the microcontroller 170 for analysis in determining whether to adjust the pacing rate, etc. Optionally, the microcontroller monitors the signals for indications of the patient's position and activity status, such as whether the patient is climbing or descending a flight of stairs, or whether the patient is sitting up after lying down.

The IMD 100 additionally includes a battery 230, which is coupled to the microcontroller 170 and is configured to provide electrical power to all of the IMD's circuits shown in Fig. 2. For the IMD, which, in this example, employs shocking therapy, the battery is capable of operating at low current drains, *e.g.*, preferably less than 10 µA, for long periods of time and is capable of providing high-current pulses for capacitor charging when the patient requires a shock pulse, *e.g.*, preferably, in excess of 2 A, at voltages above 2 V, for periods of 10 seconds or more.

The IMD 100 can further include magnet detection circuitry 232, which is coupled to the microcontroller 170, and configured to detect when a magnet (not shown) is placed over the IMD. The magnet can be used by the medical practitioner to perform various tests of the IMD and/or to signal the microcontroller that the programmer 212 is in place to receive data from, or transmit data to, the microcontroller through the telemetry circuit 222.

The IMD 100 further includes an impedance measuring circuit 234, which is coupled to the microcontroller 170 and which is enabled by the microcontroller via a control signal 236. The known uses for an impedance measuring circuit include, but are not limited to, lead impedance surveillance during the acute and chronic phases for proper lead positioning or dislodgement; detecting operable electrodes 110, 114, and 130-142 and automatically switching to an operable pair of electrodes if dislodgement occurs; measuring respiration or minute ventilation; measuring thoracic impedance for determining shock thresholds; detecting when the IMD has been implanted; measuring stroke volume; and detecting the opening of heart valves 238, etc. Advantageously, the impedance measuring circuit is coupled to the electrode configuration switch 176 so that any desired electrode can be coupled to the impedance measuring circuit.

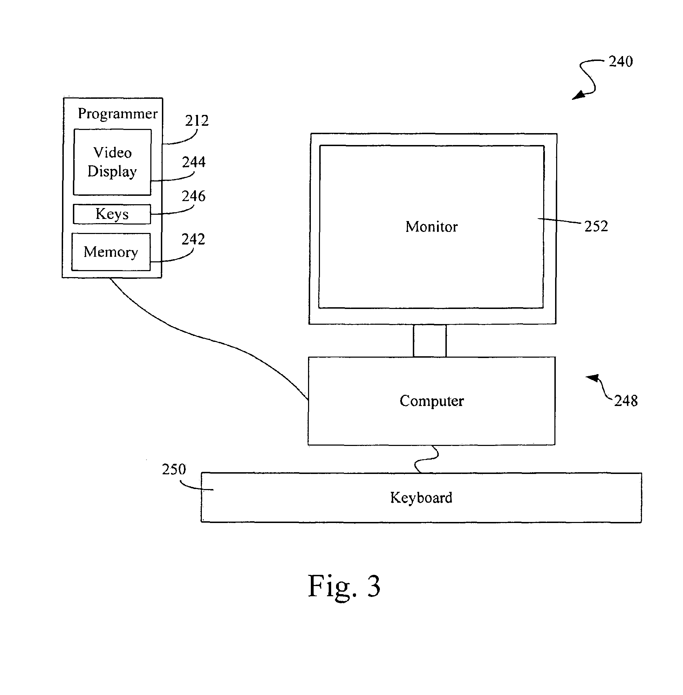

Referring additionally to Fig. 3, the programmer 212, which can be, for example, a telemetry wand or another type of communication device for wireless communication with the IMD 100, is included as part of a programmer control system 240, which is configured to communicate with the IMD. The programmer includes a programmer memory 242, which is used for storing the software used to operate the programmer, for data processing, and for long-term data storage. The programmer memory can include any type of memory suitable for long-term data storage, *e.g.*, a RAM, a ROM, an EEPROM, a flash memory, a compact disc read-only memory ("CDROM"), a digital video disc ("DVD"), a magnetic cassette, a magnetic tape, a magnetic disc drive, a rewritable optical disk, or any other medium that can be used to store information. The programmer also can include an output device 244, *e.g.*, a video display and/or a touch screen, which is configured to display data transmitted from the IMD to the programmer; and an input device 246, *e.g.*, keys and/or buttons, which is configured to receive input from the medical practitioner.

The programmer control system 240 also includes a personal computer 248, which is coupled to the programmer 212, and controls the electrical operation of the programmer. In addition, the programmer control system includes a user input device 250, *e.g.*, a keyboard, a pen, and/or a voice interface. Through the user input device, the medical practitioner can issue commands to the IMD 100 when the programmer is in communication with the IMD. The programmer control system also includes a user output device 252, *e.g.*, a monitor and/or a printer, which is coupled to the programmer and used to display the status of the IMD and/or data transmitted from the IMD to the programmer.

The medical practitioner can use the user input device 250 to prompt the transmission of information from the programmer 212 to the IMD 100, which can include IMD programming commands and interrogation commands. In response to an interrogation command transmitted from the programmer to the IMD, a wide variety of real time and stored data that is particular to the patient and to the status of the IMD can be transmitted telemetrically by the IMD, via the telemetry circuit 222, to the programmer. Also, the data transmitted from the IMD to the programmer can include information related to currently programmed IMD operating modes and parameter values, the identification ("ID") of the IMD, the patient's ID, the IMD's implantation date, the programming history of the IMD, real time event markers, and the like.

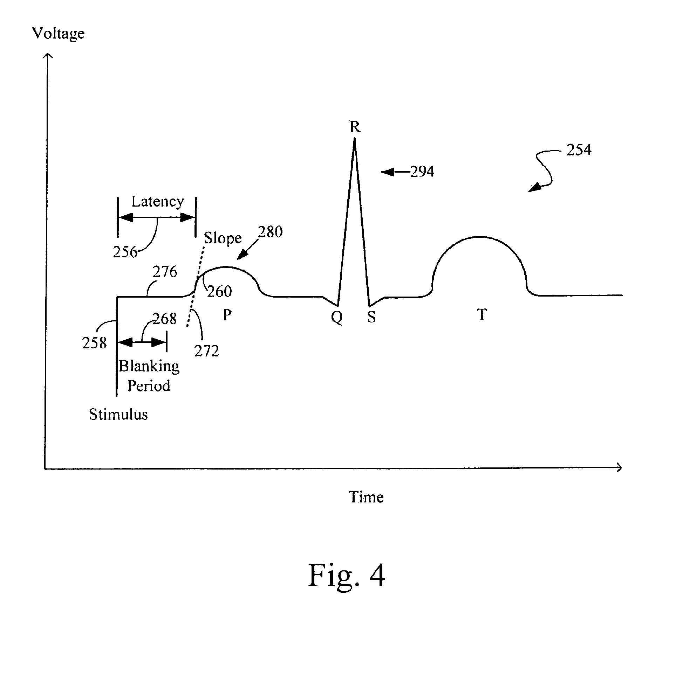

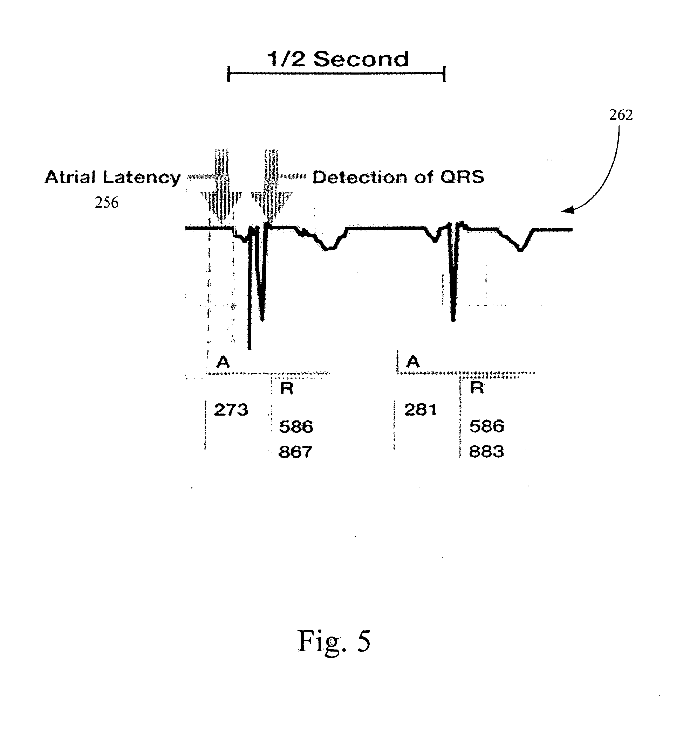

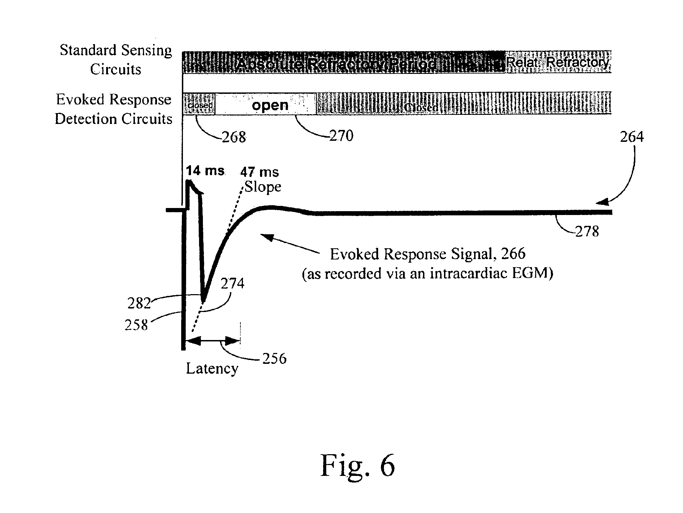

One characteristic of a patient's heart 102 that a medical practitioner can monitor using an IMD 100 in combination with a programmer control system 240 is the heart's latency. Latency in the heart is the delay measured in time, usually in milliseconds, from one event to another, *e.g.*, between the electrical stimulus of the heart and the heart's evoked response. In the field of cardiac pacing, pacing latency is a measure of the time between the delivery of an electrical stimulus from an IMD to the onset of the electrical depolarization of the myocardium in proximity to the electrode 110, 114, and 130-142 that delivered the electrical stimulus. Latency can also be measured from the time of the delivery of the electrical stimulus to a specific point in the evoked response, *e.g.*, the peak or nadir of the electrical depolarization of the chamber 112, 122, 128, and 146 of interest in the patient's heart.
Referring to Fig. 4, which is a simplified illustration of an example surface ECG 254, an example of latency 256 is the time between the electrical stimulation 258 of the heart, in this case the atrium 112, and the subsequent contraction of that muscle, *i.e.*, the time between the electrical stimulation of the atrium and the generation of the resulting P-wave 260. Another example of a surface ECG 262 that demonstrates atrial latency is shown in Fig. 5. The signal sensed by the IMD, *i*.*e*., an intracardiac EGM signal (see the example intracardiac EGM 264 shown in Fig. 6), has a different shape from that of the simplified illustration of the electrocardiogram shown in Fig. 4, as is known in the art.

As shown in Figs. 4 and 6, a latency measurement 256 is determined by measuring the time from the application of the electrical stimulus 258 to the heart 102, via the IMD 100 in combination with the leads 104-108 and their associated electrodes 110, 114, and 130-142, till the time that the evoked response signal, *e.g.*, the P-wave 260 in the surface ECG of Fig. 4, or the evoked response signal 266 in the intracardiac ECG of Fig. 6, is generated. The amount of electrical stimulation that is applied to the heart during the latency measurement is expected to be the same as the amount of electrical stimulation that typically is applied to the heart during pacing, since the latency measurement can be influenced by the amount of stimulation.
Immediately following the application of the electrical stimulus to the heart, the microprocessor 170 starts a blanking period 268 (see the "closed" period of time in Fig. 6). During the blanking period, the microprocessor turns off the atrial and/or ventricular sense amplifier 192 and 194, respectively, for a predetermined period of time to prevent the sensing of ventricular and/or atrial stimulus. After the blanking period (see the "open" period of time 270 in Fig. 6), the microprocessor turns on the atrial and/or ventricular sense amplifiers to allow for the detection of the evoked response signal using evoked response detection circuits (not shown), which are included in the tissue depolarization circuitry 188, at a time when standard sensing circuits (not shown), which also are included in the tissue depolarization circuitry, are still operating in an absolute refractory mode. During the "open" period of time, the evoked response detection circuits attempt to detect the evoked response signal.

Next, the microprocessor 170 using the electrodes 110, 114, and 130-142 senses the heart's evoked response 260 and 266 to the stimulus 258. Because the electrodes are located at different sites in the heart 102, as the number of electrodes increases, so does the potential number of latency measurements 256 and comparisons of latency measurements over time. As part of the sensing process, the microcontroller can calculate a slope 272 and 274 for Figs. 4 and 6, respectively, for the evoked response, which can be an upward slope or a downward slope. The microcontroller can use the slope to calculate the time of occurrence for the evoked response signal and to characterize the evoked response signal. In Figs. 4 and 6, the slope of the evoked response signal is positive and the latency measurement can be performed from the time of the administration of the electrical stimulation to the heart to the point in time where the slope crosses the heart's baseline response voltage level 276 and 278 in Figs. 4 and 6, respectively. The time of occurrence of the evoked response signal can be measured using other techniques, for example, the time of occurrence of the evoked response can be measured when the evoked response signal reaches a predetermined threshold value, or based on other points in the evoked response signal, *e.g.*, a peak 280 and 282 in the evoked response signal in Figs. 4 and 6, respectively. After the microcontroller determines the latency measurement, the microcontroller stores the measured latency value in memory 214. Later these pacing latency measurement values can be transmitted from the IMD 100 to the programmer 212, and displayed on the programmer's output device 244 and/or the computer's user output device 252 for viewing by the medical practitioner.

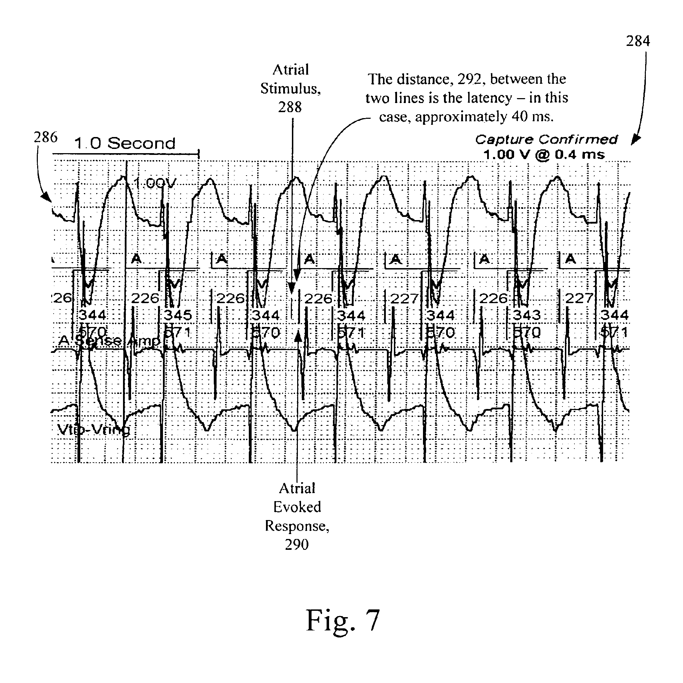

An example printout 284 of a latency measurement is shown in Fig. 7, which is a printout of an intracardiac atrial ECG 286 that illustrates a pacing latency measurement 256 determined based on the timing from an atrial stimulus 288 to an electrical depolarization of the atrium 112 and 128, *i.e.*, the atrial evoked response 290. As shown in Fig. 7, the distance 292, *i*.*e*., the length of time between the time of the atrial stimulus and the time of the atrial evoked response, is approximately 40 milliseconds.

The latency 256 that is associated with the stimulation of a muscle, *e.g.*, the heart 102, is referred to as "pacing latency." Typically, pacing latency will increase in value over time. Most commonly, an increase in pacing latency is observed in the atrium 112 and 128, however, an increase in pacing latency also can be observed in the ventricle 122 and 146. One cause of an increase in pacing latency is the slowing of the conduction of the electrical stimulating signal through the heart due to intrinsic cardiac tissue disease, which can result in myocardial dysfunction. Another cause of an increase in pacing latency is the slowing of conduction due to stretching of the heart's tissue that surrounds the electrodes 110, 114, and 130-142.

In congestive heart failure situations, particularly in the case of progressive left-ventricle dialatation and/or right-ventricle dialatation, an increase in pacing latency 256 is expected as the heart failure worsens. This is true even for cases where there is a baseline pacing latency due to scar tissue in the heart 102 at the electrode-tissue interface. In contrast, pacing latency is expected to decrease as the heart failure improves and/or the degree of dilatation of the heart decreases.

A change in the value of pacing latency 256 for the heart 102 reflects changes in the ionic dynamic of the heart's substrate, which can be correlated to heart failure status. For example, during remodeling of heart failure, *i*.*e*., the progressive decline in the heart's performance, or reverse modeling of heart failure, *i.e.*, the progressive improvement in the heart's performance, due to cardiac resynchronization therapy ("CRT"), the degree of mechanical stretch of the heart's tissue varies with the changes in the ionic dynamics of the substrate.

In this invention, pacing latency 256 is used as a surrogate marker, *i.e.*, an indicator of a physical condition, for monitoring heart failure status, which includes atrial and ventricular disease status, atrial fibrillation, myocardial dysfunction, and renal dysfunction. Pacing latency can be used in each of the left ventricle 122, right ventricle 146, left atrium 128, and right atrium 112 independently to monitor atrial or ventricular disease status. For example, pacing latency measurements taken in the left ventricle can be surrogates for left ventricular ejection fraction and/or left ventricular end-diastolic volume. Pacing latency measurements taken in the left atrium can be used to predict and/or monitor left atrium dilation, fibrosis, and the degree of mitral regurgitation, as well as progressive dysfunction.

Embodiments of the invention can include the comparison of a plurality of pacing latency measurements 256 within the same chamber 112, 122, 128, and 146 of the heart 102 over a period of time, usually measured in terms of days or weeks, but longer or shorter durations of time also are possible. This comparison can result in a trending , *i*.*e*., determining a general direction in which something moves, of the pacing latency measurements. The resulting trend can be followed prospectively which, when it crosses a threshold, triggers either a patient notification signal (discussed below) or some other method of notifying the patient and/or the medical practitioner that the heart failure is worsening before it reaches a state where it is associated with clinical symptoms. This trended data can be stored in the IMD's memory 214, and later retrieved using the programmer 212, which is configured to display, graph, print, and/or store the trended data.

In contrast to cases where pacing latency 256 is monitored for only the atrium 112 and 128 or the ventricle 122 and 146, the monitoring of pacing latency in both the atrium and the ventricle provides the medical practitioner with greater specificity of the condition of the patient's heart 102. Examples of the combination of pacing latency measurements for both the atrium and the ventricle are show in the following table:

**Table 1:**

| Left Ventricle Pacing Latency | Left Atrium Pacing Latency | Possible Clinical Outcomes |
|---|---|---|
| ↑ | ↑ | Mitral Regurgitation, Left Ventricle and/or Left Atrium Dilation |
| ↓ | ↓ | Reverse Remodeling |
| ↓ or stable | ↑ | Worsening Mitral Regurgitation but Continued Ventricular Compensation |

Table 1 indicates that when the measured value of pacing latency 256 has increased for both the left ventricle 122 and the left atrium 128, it is indicative of mitral regurgitation, left ventricle dilation, and/or left atrium dilation. In contrast, when the measured value of pacing latency has decreased for both the left ventricle and the left atrium, it is indicative of a reverse remodeling condition. When the measured value of pacing latency has decreased for the left ventricle, or remains stable, and the measured value of pacing latency for the left atrium has increased, it is indicative of worsening left atrial function such as due to progressive mitral regurgitation while ventricular function remains normal or compensated.

Pacing latency 256 also can be monitored for both atria 112 and 128, or both ventricles 122 and 146. For example, pacing latency measurements performed in both the right atrium 112 and the left atrium 128 can be used to predict atrial fibrillation remodeling in the form of left atrium dilation. In another example, pacing latency measurements performed in both the right ventricle 146 and the left ventricle 122 can be used to predict ventricular remodeling.

Pacing latency measurements 256 also can be combined with other heart failure surrogate measurements to provide the medical practitioner with greater specificity of the patient's condition and the cause of the heart failure. For example, if the measured value of pacing latency increases and the measured evoked response 260 and 266 decreases, it is more likely that the thickness of the heart's wall has decreased due to mechanical stretch/dilation. Even when the overall myocardial mass increases, mechanical stretch or dilatation of the ventricle 122 and 146 results in a net thinning of the wall. This information can be helpful to identify systolic heart failure or diastolic heart failure conditions. With pressure overload and myocardial mass increases, an increase in the measured evoked response along with an increase in the measured pacing latency can indicate that the heart failure status is not improving. An example of the combination of pacing latency measurements for the left ventricle 122 in combination with evoked response measurements taken for the left ventricle are shown in the following table:

**Table 2:**

| Left Ventricle Pacing Latency | Left Ventricle Evoked Response | Possible Clinical Outcomes |
|---|---|---|
| ↑ | ↓ | Left Ventricle Wall Thickness ↓; Left Ventricle Dilation; Systolic Heart Failure |
| ↑ | ↑ | Left Ventricle Pressure and Left Ventricle Wall Thickness ↑; Diastolic Heart Failure |
| ↓ | ↓ | Reverse Remodeling Effect |

Table 2 indicates that when the measured value of pacing latency 256 has increased for the left ventricle 122 and the measured value of evoked response 294 for the left ventricle has decreased in value, the thickness of the left ventricle wall has decreased, there has been a dilation of the left ventricle, and/or systolic heart failure has occurred. Table 2 also indicates that when the measured values of pacing latency and evoked response for the left ventricle both have increased, that left ventricle pressure and left ventricle wall thickness have increased. Also, when the measured values of left ventricle pacing latency and evoked response both have increased it can be indicative of diastolic heart failure. In contrast, when the measured values of left ventricle pacing latency and evoked response both have decreased, it is indicative of a reverse remodeling effect.

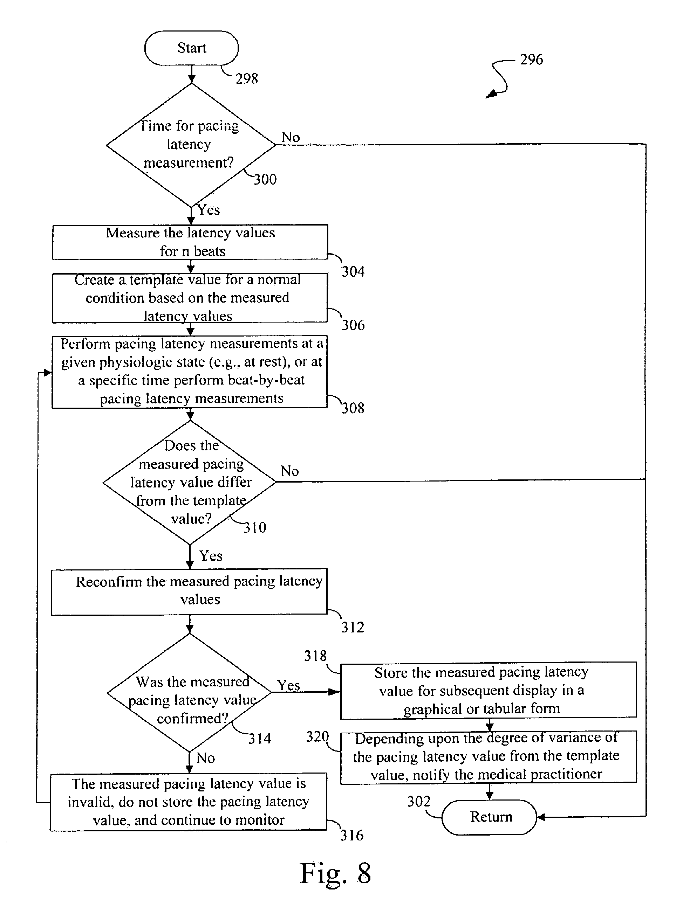

Fig. 8 is a flowchart that shows an example algorithm 296 for performing measurements of pacing latency values 256 that is implemented using the microcontroller 170. The algorithm starts at step 298. Next, at step 300, the microcontroller determines if a measurement of a pacing latency value 256 should be performed. If not, then the next step 302 is to return to the start of the algorithm.

If the microcontroller 170 determines that a pacing latency measurement 256 should be performed, then, at step 304, the microcontroller measures the latency values for n-beats, where n is an integer value greater than one that can be preset. Next, at step 306, the microcontroller establishes a template value, *e.g.*, an average pacing latency value, for a normal condition based on the measured pacing latency. At step 308, the microcontroller performs pacing latency measurements. The pacing latency measurements can be performed while the patient is in a specific physiologic state, *e.g.*, at rest, during mild or moderate exercise levels, which could be defined by a specific sensor input response, when the patient is supine, or at a specific time. Also, the pacing latency measurements can be measured in similar physiologic states over successive days, weeks, or months to develop trends, even when the patient's condition is considerer abnormal, which can be used to detect a worsening or improvement in the patient's heart failure.

At step 310, the microcontroller 170 determines if the measured pacing latency value 256 is different from the template value, which, as noted above, is a previously measured pacing latency value. If not, then the next step of the algorithm is the return step 302. At step 312, if the measured pacing latency value is different from the template value, then the microcontroller reconfirms the pacing latency measurements by remeasuring the pacing latency value and recomparing that measurement to the initial pacing latency measurement. If the pacing latency measurements are not confirmed at step 314, then the microcontroller considers the pacing latency measurements invalid at step 316, and the microcontroller repeats the pacing latency measurement at step 308. If the pacing latency measurements are confirmed, then, at step 318, the measured pacing latency value is stored in memory 214 for later display on the user output device 244 and 252, and, depending upon the degree of variance of the measured pacing latency value from the template value, the medical practitioner is notified on the user output device, as indicated in step 320. Thus, the medical practitioner can be notified of the change in the amount of cardiac disease in a region of the heart 102 based on a relative change in the measured pacing latency value, or an absolute change in the measured pacing latency value. For example, if the measured pacing latency value is 20 milliseconds at baseline when the patient is clinically stable and increases to 30 milliseconds, or greater than a 50 percent increase from the template baseline value, then a message is displayed on the user output device for the medical practitioner. Thus, the microcontroller can issue a warning message for viewing by the medical practitioner when there is a possibility of clinical concerns.

Accordingly, the medical practitioner can be notified of an absolute change in the measured pacing latency value 256 or a relative change in the measured pacing latency value. Also, the medical practitioner can be notified of the absolute or relative change in the measured pacing latency value when the change in the measured pacing latency value is greater than or equal to a predetermined threshold value. In embodiments of the invention, the warning message can be displayed on the programmer's output device 244 for viewing by the medical practitioner, or the computer's user output device 252. In other embodiments, the IMD 100 is configured to generate a patient notification signal, which notifies the patient when the IMD's microcontroller 170 determines that the absolute change or the relative change in the measured pacing latency value is greater than or equal to the predetermined threshold value. Example patient notification methods are described in U.S. Patent No. 6,546,288 to Levine, which is incorporated by reference herein.

As noted above, pacing latency measurements 256 can be combined with other heart failure surrogate measurements to provide the medical practitioner with a more specific diagnosis of the heart's condition. If heart failure surrogate measurements are included, the algorithm 296 can be modified to include the measurement and consideration of one or more of these additional heart failure surrogates.

Pacing latency measurements 256 can be implemented using existing heart monitoring systems, *e.g.*, the AUTOCAPTURE Pacing Systems offered by Pacesetter of Sylmar, California. Using a heart monitoring system, the time delay from the application of an electrical stimulation pulse 258 to its associated evoked response 260 and 266 easily can be measured and a daily average of captured beats can be stored in the IMD's memory 214 for comparison purposes (in the algorithm 296 illustrated in Fig. 8, the daily average of capture beats can be the template value). The previous discussed algorithm and related pacing latency measurement techniques can be embodied in a computer-readable medium, *e.g.*, the IMD's memory that includes instructions for the microcontroller 170.

An advantage that is associated with the use of pacing latency values 256 to monitor heart failure status is that pacing latency measurements are not influenced by the patient's posture. Pacing latency measurements are not posture dependent because each latency measurement is determined from the time delay between a stimulus pulse 258 and its associated evoked response 260 and 266. In contrast, a patient's posture is a concern during evoked response and impedance measurements because they are amplitude measurements. Another advantage that is associated with pacing latency measurements is that pacing latency measurements can be easily and inexpensively obtained using algorithms for measuring evoked response in existing heart monitoring systems.

Although the present invention is described herein in conjunction with an IMD 100 having a microprocessor-based architecture, it will be understood that the IMD and the previously discussed algorithm 296 can be implemented using any logic-based, custom, integrated circuit architecture, if desired. Also, while the previous discussion has focused on the insertion of the IMD leads 104-108 into a heart chamber 112, 122, 128, and 146 via the patient's venous system (not shown), it should be understood by those individuals having ordinary skill in the art that one or more of the IMD leads can be coupled externally to the patient's heart 102. In such a case, the IMD lead is inserted into the patient's chest cavity (not shown) through a hole (not shown) in the pericardium (not shown), and the lead is secured to the heart's epicardial tissue 322 (see Fig. 1).

## Claims

1. An implantable medical device (100) comprising: a microcontroller (170) that is adapted to measure a pacing latency value in a region of a heart (102), and to determine a cardiac disease condition in the region of the heart (102) based on a comparison of the measured pacing latency value to a previously measured pacing latency value.

2. A device as claimed in Claim 1, **characterised in that** the microcontroller (170) is configured to measure a heart failure surrogate value, and to characterise an amount of cardiac disease for the heart based on a combination of the measured heart failure surrogate value and the comparison of the measured pacing latency values.

3. A device as claimed in Claim 1 or Claim 2, **characterised in that** the implantable medical device (100) is configured to notify a medical practitioner of the change in the amount of cardiac disease in the region of the heart (102).

4. A device as claimed in Claim 3, **characterised in that** the medical practitioner is notified of the change in the amount of cardiac disease in the region of the heart (102) when an absolute change in the measured pacing latency value or a relative change in the measured pacing latency value is greater than or equal to a predetermined threshold value.

5. A system comprising: means for measuring a pacing latency value for a region of a heart (102); means for comparing the measured pacing latency value to a previously measured pacing latency value for the region of the heart (102); and means for determining a cardiac disease condition based on the comparison of the measured pacing latency values.

6. A system as claimed in Claim 5, **characterised by** means for generating a notification signal of the cardiac disease condition.
